# EUROPEAN PATENT APPLICATION

(11) **EP 1 952 796 A1**
(43) Date of publication of application: **06.08.2008**
(21) Application number: 06018843.0
(22) Date of filing: 08.09.2006
(51) Int. Cl.: A61K 8/34, A61K 8/36, A61K 8/37, A61K 8/49, A61K 8/31, A61Q 19/08

(54) **Skin care compositions**

(71) Applicant: DSM IP Assets B.V., 6411 TE Heerlen (NL)
(72) Inventor: The designation of the inventor has not yet been filed
(74) Representative: Schwander, Kuno

(57) **Abstract**

The present invention relates to an oral composition containing an optimal combination of nutritional ingredients at high concentrations and at optimal ratios in order to maintain and/or achieve health and/or beautiful look of the skin.

## Description

The present invention relates to an oral composition containing an optimal combination of nutritional ingredients at high concentrations and at optimal ratios in order to maintain and/or achieve health and/or beautiful look of the skin.

The skin is the largest organ of the body. It is made up of multiple layers of epithelial tissues that guard underlying muscles and organs. As the interface with the surroundings, it plays the most important role in protecting the body against pathogens. It furthermore provides sensory perception, moisture control, shape, regulation of metabolic processes, immune response and insulation against heat and cold. The skin also has the remarkable role of defining our individuality by transmitting information back to the environment about a person's appearance, behavior and health.

The outermost layer of the skin is called epidermis. It forms the waterproof, protective wrap over the body's surface and is made up of stratified squamous epithelium with an underlying basal lamina. It contains no blood vessels, and is nourished by diffusion from the dermis. The main types of cells which make up the epidermis are keratinocytes, with melanocytes and Langerhans cells also present. Epidermis is divided into several layers where cells are formed through mitosis at the innermost layers. They move up the strata changing shape and composition as they differentiate and become filled with keratin. They eventually reach the top layer called stratum corneum and become sloughed off, or desquamated. This process is called keratinization and takes place within weeks. The outermost layer of Epidermis consists of 25 to 30 layers of dead cells. Every day thousands of new skin cells are generated.

The body naturally looses water by constant gentle evaporation through the skin (transepidermal water loss, TEWL). Preventing excessive water loss is exceptionally important in itself - both to the skin itself and to the body as a whole. In the normal epidermis the water content gets less the closer we get to the surface. Water makes up to 70 to 75 % of the weight of the basal layer, but only 10 to 15 % of the stratum corneum. The presence of an adequate amount of water in the epidermis is important for the general appearance of a soft, smooth and attractive skin.

The stratum corneum is hydrated by the water trapped in lower layers as well as by normal perspiration. If it is deficient in natural moisturising factors or subjected to extreme weather conditions, the skin loses moisture. It becomes dry and taut. As it becomes less supple, its protective function is reduced. The skin chaps, transepidermal water loss increases, and its barrier function is impaired. As the skin ages, sebum (or oil) production slows down. Production of moisture-binding substances also decreases. The skin becomes drier and less able to retain moisture. It takes on a flat, dull appearance and is less smooth. Fine lines and wrinkles begin to form and the skin is less firm.

There is a multitude of different intrinsic and external factors that influence skin condition and foster skin aging. Intrinsic factors are genetic predisposition, the normal aging process, or hormonal status. UV radiation, air pollution, smoking and/or allergenic compounds are external factors which can account for premature skin aging. As the skin ages chronically it particularly loses elasticity. Chronic exposure to UV radiation (UVB and UVA) leads premature skin aging through epidermal and dermal damage by oxidative stress and sunburn. Sunburn is the inflammatory reaction of the skin in response to excessive exposure to natural or artificial solar light of the UVB wavelength.
Hyperkeratosis, keratinocyte dysplasia and/or dermal elastosis occur in affected skin areas, clinically presenting as photoaged skin with actinic or solar keratosis. These precancerous lesions show an increased risk for the development of squamous cell carcinoma (SCC). The clinical condition of premature skin aging is accompanied with hyperpigmentation and dilated and twisted microvasculature, i.e. teleangiectasia.

In addition to other influencing factors also nutrition has a strong impact on the skin's condition. Optimization of the diet improves general skin condition and hydration of the skin. A deficiency in essential vitamins and/or fatty acids e.g. has clear cutaneous effects. Therefore for full skin protection, a well-balanced mix of different nutritional substances is essential. Optimally, micronutrients, i.e. vitamins, certain polyunsaturated fatty acids (PUFAs) and other nutritional active compounds such as antioxidants are supported internally, i.e. by oral administration, absorption, and transport to the skin via the blood stream.

PUFAs are known to be important for the preservation of the skin-barrier function and the water content of the skin. They are incorporated into the cell membranes of skin keratinocytes and fibroblasts. Due to their structure, PUFAs enhance membrane fluidity, and thus contribute to more flexible and mobile cell membranes than saturated fatty acids. Oral intake of certain PUFAs such as gamma-linolenic acid or linoleic acid leads to a more moistened and smoother skin.

The present invention relates to an oral composition providing an optimal combination of nutritional ingredients at high concentrations and at optimum ratios, preferably in capsules whereas the composition should show excellent bioavailability.

The present invention relates to a new formula for skin health containing polyunsaturated fatty acids and at least one compound selected from polyphenols and/ or lipid soluble and water soluble vitamins, co-enzymes, anti-oxidants, carotenoids and their esters.

In a preferred embodiment the present invention relates to a composition for oral intake comprising
i) at least a compound selected from the group consisting of polyunsaturated fatty acids, esters of polyunsaturated fatty acids such as ethyl esters, mono-, di- and triglyceridesters, and fish oil; and
ii) at least a compound selected from the group consisting of carotenoids, water-soluble vitamins, fat-soluble vitamins, ubichinones and polyphenols,
whereas the total amount of component(s) ii) in the composition is at least 40 weight-%, preferably at least 50 weight-%, based on the total weight of component(s) i) and based on the weight of the pure compounds.

In an especially preferred embodiment of the present invention the components i) are n-3 polyunsaturated fatty acids and derivatives thereof. Preferred examples of n-3 polyunsaturated acids are eicosapenta-5,8,11,14,17-enoic acid (EPA) and docosahexa-4,7,10,13,16,19-enoic acid (DHA), as well as arachidonic acid (ARA).
Preferred derivatives of n-3 polyunsaturated fatty acids are their ethyl esters as well as their mono-, di- and triglycerides. Triglycerides of n-3 polyunsaturated fatty acids are especially preferred. When the derivatives are triglycerides, normally three different n-3 polyunsaturated fatty acids are esterified with glycerin. Preferred triglycerides may also partly contain saturated fatty acids. In one preferred embodiment of the present invention triglycerides are used, whereby 30 % of the fatty acid part are n-3 fatty acids and of these 25 % are long-chain polyunsaturated fatty acids. In a further preferred embodiment commercially available ROPUFA® '30' n-3 Food Oil (DSM Nutritional Products Ltd, Kaiseraugst, Switzerland) is used.
Alternatively other polyunsaturated fatty acids (omega-3 fatty acids; omega-6 fatty acids) and/or their derivatives may be used.
Most preferably, the PUFAs are esters of the above defined PUFAs. One example for such a PUFA ester is ROPUFA®'75' n-3 EE.
ROPUFA '75' n-3 EE is a refined marine oil in form of an ethyl ester with minimum content of 72 % n-3 fatty acid ethyl ester. It is stabilized with mixed tocopherols, ascorbyl palmitate, citric acid and contains rosemary extract.

In a preferred embodiment of the present invention the components ii) to be used in compositions according to the invention are the water soluble vitamins C, B₆, B₂,, B₁₂, biotin, calcium-pantothenate and pharmaceutically acceptable salts thereof; the fat (lipid) soluble vitamin A, vitamin E and pharmaceutically acceptable salts and derivatives thereof, the ubichinones coenzyme Q 10 and vitamin K and pharmaceutically acceptable derivatives thereof, the polyphenols epigallocatechin galate (EGCG), hydroxytyrolsol, olive extract, resveratrol, genistein and pharmaceutically acceptable derivatives thereof, and the carotenoids (comprising the group of xanthophylls) beta-carotene, lycopene lutein, zeaxanthin and their esters.

The composition according to the present invention is most suitable to support healthy skin appearance and beauty. It supports skin nourishment from inside the body via the blood stream (systemically), fosters hydration and moisturizes the skin, supports skin barrier function, provides protection against oxidative stress and UV-radiation and has a general anti-aging effect. Thus, it supports beauty from inside. The composition also promotes skin repair and regeneration upon healing of injuries as well as the physiological renewal process.

Overall the composition is promoting an optimal health, a natural radiance and glow and/or a beautiful look of the skin. According to the present invention the term "beautiful look of the skin" comprises a good blood supply generating a natural pink tone, a natural radiance and glow, a pure, clear appearance lacking major impurities and normal sebum secretion, good elasticity, hydration and moisture of the skin and an intact skin barrier function.

Optimal skin moisture and hydration means little transepidermal water loss and intact skin barrier function. Well-hydrated skin is more firm and toned. Hydration minimizes the appearance of fine lines and wrinkles.
The hydration level of the outermost skin layer (10-20 µm depth), the stratum corneum, can be measured with a corneometer. The corneometric measurement allows an interpretation about the condition of the skin, the skin type and the effects of pharmaceutical and cosmetic products.
The principle of the Corneometer is based on a capacitance measurement of a dielectric medium. Water has a relatively high dielectric constant (D = 80) in contrast to most other substances (D < 7). Even slightest changes in skin surface hydratation alters the dielectric constant and therefore the capacitance of a precision measuring capacitor. A spring in the probe head ensures constant pressure of 3,5 N on the skin. The measuring surface is 49 mm². The reproducibilty and accuracy (± 3%) of the measurement is high and the measurement time is 1 s only (prevents occlusion effects).
Boosting of moisture-binding capability of skin enables it to retain its healthy glow. Super hydrated skin is softer to the touch and has a smoother appearance.

In the event of an injury that damages the skin's protective barrier, the body triggers a response called inflammation, which sends fluids carrying phagocytic white blood cells to the injury site. Once the invading microorganisms have been brought under control, the skin proceeds to heal itself. The ability of the skin to heal even after considerable damage has occurred is due to the presence of stem cells in the dermis and cells in the stratum basale of the epidermis, all of which can generate new tissue.
In healthy skin, the immune defense is intact, inflammation within physiological range and the self healing process functional. The composition as defined above can support skin repair e.g. by occlusion, by partly replacing the lipids or by triggering cellular lipid production.

Epidermal keratinocytes are produced constantly from stem cells in the basal layer of the epidermis. During their migration upwards, the keratinocytes run through a differentiation process. The result of epidermal differentiation is the formation of the stratum corneum, which consists of terminally differentiated, cornified keratinocytes. The process from proliferation to desquamation takes about one month. The composition as defined above supports this renewal process.

### Resveratrol

The term "resveratrol" as used herein comprises resveratrol itself and derivatives, metabolites or analogues thereof. The carbon-carbon double bond may be trans or cis and includes cis/trans mixtures. Etherified or esterified hydroxy groups may be derived from unsubstituted or substituted, straight or branched chain alkyl groups having 1 to 26 carbon atoms or from unsubstituted or substituted, straight or branched chain aliphatic, araliphatic or aromatic carboxylic acids having 1 to 26 carbon atoms. Etherified hydroxy groups may further be glycoside groups and esterified hydroxy groups may further be glucuronide or sulfate groups. Of primary interest for the purposes of the invention is (trans)-resveratrol.

### Genistein

The term "genistein" as used herein comprises the aglycone (4', 5, 7-trihydroxyisoflavone) and derivatives thereof, e.g., genistein glycosides, genistein sulfates, genistein glucuronides.

### (-)-Eoigallocatechin gallate and derivatives thereof

The term "(-)-epigallocatechin gallate" (EGCG) encompasses also green tea extracts containing (-)-EGCG as well as (-)-EGCG derivatives such as pharmaceutically acceptable salts.

An especially suitable (-)-EGCG is e.g. Teavigo (a green tea extract containing ≥ 94% of EGCG), commercially available from DSM Nutritional Products Ltd, Kaiseraugst, Switzerland, as well as Teavigo TG (Tablet Grade) (a green tea extract containing ca. 88% of EGCG admixed with ca. 3% of pectin).

A preferred alternative for (-)-epigallocatechin gallate is a green tea fraction comprising at least 85.0 % weight-% of (-)-epigallocatechin gallate (EGCG) and at most 2.0 weight-% of caffeine, especially a green tea fraction comprising at least 90.0 weight-% of (-)-epigallocatechin gallate (EGCG) and at most 1.6 weight-% of caffeine, whereas this green tea fraction preferably comprises at most 4.0 weight-% of epicatechin (EC), and/or at most 4.0 weight-% of catechin, and/or at most 2.0 weight-% of gallocatechin gallate (GCG), and/or at most 5.0 weight-% of epicatechin gallate (ECG).

A preferred alternative for (-)-epigallocatechin gallate is a green tea fraction comprising at least 91.7 weight-% of (-)-epigallocatechin gallate (EGCG) and at most 1.43 weight-% of caffeine, especially a green tea fraction comprising from 91.7 to 97.13 weight-% of EGCG, from 0 to 3.15 weight-% of epicatechin (EC), from 0 to 3.1 weight-% of catechin, from 0.2 to 1.52 weight-% of gallocatechin gallate (GCG), from 0.38 to 4.62 weight-% of epicatechin gallate (ECG) and from 0 to 1.43 weight-% of caffeine.

### Hydroxytyrosol and derivatives thereof

The term hydroxytyrosol relates to 'pure hydroxytyrosol' of either synthetic origin or obtainable from natural sources such as from products and by-products derived from the olive tree by extraction and/or purification. It may also be obtained together with other water-soluble polyphenols such as tyrosol and oleuropein in the form of an olive extract. Additionally the term hydroxytyrosol encompasses hydroxytyrosol comprising extracts obtainable e.g. from products and by-products derived from the olive tree.

Products and by products of olive trees encompass olives, olive tree leafs, olive pulps, olive oil, olive-derived vegetation water and olive oil dregs without being limited thereto. Based on the extraction procedure the amount, respectively the ratio of the hydroxytyrosol can be easily adjusted by a person skilled in the art. Preferably, hydroxytyrosol is derived from olives that may be obtained from conventional and commercially available sources such as growers.

Examples of references that deal with the extraction hydroxytyrosol and/ or oleuropein from olive leaves are WO02/18310 A1, US 2002/0198415 A1, WO2004/005228 A1, US 6,416,808 and US 2002/0058078 A1 which disclose a method for acidic hydrolysis of olive vegetation water for 2 to 12 months until at least 90% of the present oleuropein has been converted. A method of extraction of phenolic compounds from olives, olive pulps, olive oil and oil mill waste water is described by Usana Inc. patents US 6,361,803 and WO01/45514 A1 and in US 2002/0004077 A1. EP 1 582 512 A1 describes an extraction of hydroxytyrosol from olive leaves. A method for obtaining hydroxytyrosol from the vegetation water of de-pitted olives is disclosed in US 2004/0039066 A1 in paragraphs [0080]-[0091]."

Preferably hydroxytyrosol is used in the form of a hydroxytyrosol containing olive extract.

Commercially available hydroxytyrosol containing olive extracts which may be used according to the invention include e.g. extracts from olive fruits such as Polyphen-Oil™ from Life Extension, OleaSelect™ from Indena, Hytolive^{®} from Genosa, Prolivols from Seppic, OLIVE LEAF or OLIVE Water Extract of Olea europea from Lalilab, Hitofulvic from Ebiser, hydrolysed olive leaf extract, such as described in EP1582512, olive leaf extract, rich in oleuropein, such as available from Furfural and HIDROX^{®} from CreAgri.
Preferably HIDROX^{®} from CreAgri such as HIDROX^{®} 2% spray dried powder, HIDROX^{®} Gold freeze dried powder (9%) and HIDROX^{®} 6% freeze dried powder organic olive juice extract are used.

Derivatives may be esters as well as physiologically/pharmaceutically acceptable salts.

### Lycopene and derivatives thereof

The term "lycopene" includes all-E and Z-stereoisomers. Alternatively a tomato extract which contains high amounts of lycopene could also be used.

### Lutein and derivatives thereof

The term "lutein" includes all-E and Z-stereoisomers. Suitable derivatives are e.g. its mono-and di-esters, preferably esters of saturated alkanoic acids such as acetic, propionic, laurinic, myristinic, palmitic, stearic and succinic acid, esters of mono-unsaturated fatty acids such as oleic acid, and poly-unsaturated fatty acids such as linolic, linoleic, pentaenoic, docosahexaenoic and arachidonic acid, and mixtures thereof.

### Zeaxanthin and derivatives thereof

The term "zeaxanthin" includes all-E and Z-stereoisomers. Suitable derivatives are e.g. its mono-and di-esters, preferably esters of saturated alkanoic acids such as acetic, propionic, laurinic, myristinic, palmitic, stearic and succinic acid, esters of mono-unsaturated fatty acids such as oleic acid, and poly-unsaturated fatty acids such as linolic, linoleic, pentaenoic, docosahexaenoic and arachidonic acid, and mixtures thereof.

The effective amounts of the active ingredients as listed above may preferably be selected in all embodiments of the invention in the following ranges:
PUFA(s), in particular n-3 polyunsaturated fatty acids and/or its derivatives (especially triglycerides): daily dosage for humans (70 kg person): from 50 mg to 8 g, preferred daily dosage for humans (70 kg person) from 300 mg to 2000 mg.
Resveratrol: daily dosage for humans (70 kg person): 1 to 100 mg, preferred daily dosage for humans (70 kg person): 5 to 50 mg, more preferred from 20 to 30 mg.
Genistein: is daily dosage for humans (70 kg person): 1 to 150 mg, preferred daily dosage for humans (70 kg person) 20 to 60 mg, more preferred from 20 to 40 mg.
(-)-Epigallocatechin gallate: daily dosage for humans (70 kg person): 50 to 600 mg, preferred daily dosage for humans (70 kg person): 150 to 300 mg.
Hydroxytyrosol: daily dosage for humans (70 kg person) in pure form: 0.25 to 500 mg, preferred daily dosage for humans (70 kg person): 50 to 100 mg. Alternatively, olive extract according the definition above: 10 mg to 500 mg, more preferably 50 to 400 mg, and more preferred 100 to 200 mg.
β-Carotene: daily dosage for humans (70 kg person): 0.1 to 50 mg, preferred daily dosage for humans (70 kg person): 1 and 30 mg, more preferred daily dosage for humans (70 kg person): 2 to 7 mg.
Lycopene: For usual applications the daily dosage for humans (usually determined for a 70 kg person) for lycopene should not exceed 40 mg, preferably not exceed 25 mg. In some embodiments of the invention the daily dosage for humans (70 kg person) for lycopene can be between 0.1 to 40 mg, more preferably between 0.5 to 25 mg.
Lutein: For usual applications the daily dosage for humans (usually determined for a 70 kg person) for lutein not exceed 60 mg, preferably not exceed 30 mg.

In some embodiments of the invention the daily dosage for humans (70 kg person) for lutein between 0.1 to 60 mg, more preferably between 1.0 to 30 mg.
Zeaxanthin: daily dosage for humans (70 kg person): 0.1 to 20 mg, preferred daily dosage for humans (70 kg person): 2 to 7 mg, more preferred daily dosage for humans (70 kg person): ca. 4 mg.
Biotin: daily dosage for humans (70 kg person): 10 µg to 5 mg, preferred daily dosage for humans (70 kg person): 20 µg to 2 mg, more preferred daily dosage for humans (70 kg person): 30 µg to 500 µg
Vitamin E: For humans (70 kg person) the daily dosage preferably may vary for vitamin E between 10 mg and 2 g, more preferably between 15 and 500 mg.
Vitamin C: For humans (70 kg person) the daily dosage preferably may vary for vitamin C between 50 mg and 5 g, more preferably between 200 mg and 1.5 g.
Vitamin B12: daily dosage for humans (70 kg person): 0.5 to 10 µg, preferred daily dosage for humans (70 kg person): 1 to 5 µg, more preferred daily dosage for humans (70 kg person): 2.4 to 3 µg.
Vitamin B6: daily dosage for humans (70 kg person): 0.1 to 20 mg, preferred daily dosage for humans (70 kg person): 1 to 10 mg, more preferred daily dosage for humans (70 kg person): 2.0 to 6 mg.
   Vitamin B2 daily dosage for humans (70 kg person): 0.1 to 20 mg, preferred daily dosage for humans (70 kg person): 0.5 to 15 mg, more preferred daily dosage for humans (70 kg person): 1.0 to 10 mg.
Ca-D-Pantothenate daily dosage for humans (70 kg person): 0.5 to 30 mg, preferred daily dosage for humans (70 kg person): 1 to 20 mg, more preferred daily dosage for humans (70 kg person): 2.0 mg to 10 mg.
CoQ-10: daily dosage for humans (70 kg person): 1 to 100 mg, preferred daily dosage for humans (70 kg person): 5 to 60 mg.

If instead of 'pure active ingredients' an active ingredient comprising extract is used, the amount of the extract to be used may be derived from the concentration of the 'pure active ingredient' within the extract and the finding of the optimal dosage is a matter of routine experimentation for the person skilled in the art.

In all embodiments of the invention the definitions and suitable daily dosages for the active ingredients to be used according to the invention as described above apply.

The daily amounts for the compounds used in accordance with the invention indicated may be applied in a single dosage or in multiple dosages.

The invention also relates to a composition for consumption by humans comprising
a) at least a compound selected from the group consisting of polyunsaturated fatty acids, esters of polyunsaturated fatty acids such as ethyl esters, mono-, di- and triglyceridesters, and fish oil, in amount in the range of from 0.50 mg to 120 mg per kg bodyweight of said humans, preferably from 5 mg to 30 mg per kg bodyweight of said humans; and at least one of the components b) to k),
b) β-carotene, in an amount in the range of from 0.0014 to 0.7 mg per kg bodyweight, preferably from 0.01 and 0.5 mg per kg bodyweight of said humans;
c) lycopene, preferably in an amount in the range of from 0.0014 to 0.6 mg, more preferably between 0.007 to 0.35 mg per kg bodyweight of said humans;
d) lutein, in an amount in the range of from 0.0014 to 0.86 mg, preferably between 0.014 to 0.5 mg per kg bodyweight of said humans;
e) zeaxanthin, in an amount in the range of from 0.0014 to 0.28 mg, preferably between 0.028 to 0.1 mg per kg bodyweight of said humans;
f) vitamin E, in an amount in the range of from 0.14 mg and 30 g, preferably between 0.2 and 7 mg per kg bodyweight of said humans;
g) vitamin C, in an amount in the range of from 0.71 mg and 70 g, preferably between 2.5 mg and 20 mg per kg bodyweight of said humans;
h) epigallocatechin gallate, in an amount in the range of from 0.5 to 8.5 mg, preferably 2.0 to 4.3 mg per kg bodyweight of said humans; and/or
i) hydroxytyrosol, in an amount in the range of from 0.071 to 7.1 mg, preferably 0.71 to 1.42 mg per kg bodyweight of said humans;
j) genistein: in an amount in the range of from 0.014 to 2.14 mg, preferably 0.28 to 0.85 mg per kg bodyweight of said humans;
k) resveratrol: in an amount in the range of from 0.014 to 1.4 mg, preferred preferably 0.071 to 0.71 mg, more preferred from 0.28 to 0.42 mg per kg bodyweight of said humans.
l) biotin, in an amount in the range of from 0.14 µg to 71 µg, preferably 0.28 µg to 28 µg per kg bodyweight of said humans;
m) coenzyme Q 10, in an amount in the range of from 0.014 to 1.4 mg, preferably 0.071 to 0.85 mg per kg bodyweight of said humans;
wherein the total amount of components b) to j) is at least 40 weight-%, preferably at least 50 weight-%, based on the total weight of component(s) a) and based on the weight of the pure compounds.

Preferably, the invention relates to a composition for consumption by humans comprising
i) at least a compound selected from the group consisting of polyunsaturated fatty acids, esters of polyunsaturated fatty acids such as ethyl esters, mono-, di- and triglyceridesters, and fish oil, in amount in the range of from 0.50 mg to 120 mg per kg bodyweight of said humans, preferably from 5 mg to 30 mg per kg bodyweight of said humans; and
ii) at least a polyphenol selected from the group consisting of epigallocatechin gallate, genistein, resveratrol and pharmaceutically acceptable derivatives thereof, in an amount in the range of from 0.014 to 8.5 mg, preferably 0.14 to 4.3 mg per kg bodyweight of said humans; and/or hydroxytyrosol and pharmaceutically acceptable derivatives thereof, in an amount in the range of from 0.014 to 7.1 mg, preferably 0.71 to 1.42 mg per kg bodyweight of said humans,
   and/or olive extract in an amount in the range of from 0.014 to 7.1 mg, preferably 0.71 to 1.42 mg per kg bodyweight of said humans;

In a even more preferred embodiment the composition for consumption by humans comprising at least a compound selected from the group consisting of polyunsaturated fatty acids, esters of polyunsaturated fatty acids and at least a polyphenol comprises an additional amount of lipid soluble and/ or water soluble vitamins, co-enzymes, anti-oxidants, carotenoids and/ or their esters with the definitions and preferences outlined above.

Furthermore, the invention relates to the use of the compositions according to the invention for supporting a healthy skin appearance and beauty, for supporting skin nourishment from inside the body via the blood stream (systemically), for fostering hydration, for moisturizing the skin, for supporting the skin barrier function, for providing protection against oxidative stress, for providing protection against UV-radiation and/or for providing a general anti-aging effect., in particular for promoting skin repair and/or regeneration upon healing of injuries and/or for promoting the physiological renewal process and thus for promoting an optimal health, a natural radiance and glow and/or a beautiful look of the skin.

In another embodiment the invention relates to a method of administering an effective amount of
i) at least a compound selected from the group consisting of polyunsaturated fatty acids, esters of polyunsaturated fatty acids such as ethyl esters, mono-, di- and triglyceridesters, and fish oil; and
ii) at least a compound selected from the group consisting of carotenoids, water-soluble vitamins, fat-soluble vitamins, ubichinones and polyphenols,
whereas the total amount of component(s) ii) in the composition is at least 40 weight-%, preferably at least 50 weight-%, based on the total weight of component(s) i) and based on the weight of the pure compounds to humans in order to support a healthy skin appearance and beauty, to support skin nourishment from inside the body via the blood stream (systemically), to foster hydration, to support moisturizing the skin, to support the skin barrier function, to provide protection against oxidative stress, to provide protection against UV-radiation and/or to provide a general anti-aging effect, in particular to promote skin repair and/or regeneration upon healing of injuries and/or to promote the physiological renewal process and thus to promote an optimal health, a natural radiance and glow and/or a beautiful look of the skin.

The term 'an effective amount' refers to an amount necessary to obtain a physiological effect. The physiological effect may be achieved by one single dose or by repeated doses. The dosage administered may, of course, vary depending upon known factors, such as the physiological characteristics of the particular composition and its mode and route of administration; the age, health and weight of the recipient; the nature and extent of the symptoms; the kind of concurrent treatment; the frequency of treatment; and the effect desired and can be adjusted by a person skilled in the art. The necessary daily amounts may be applied in a single dosage or in multiple dosages.

The at least a compound selected from the group consisting of polyunsaturated fatty acids, esters of polyunsaturated fatty acids such as ethyl esters, mono-, di- and triglyceridesters, and fish oil; and at least a compound selected from the group consisting of carotenoids, water-soluble vitamins, fat-soluble vitamins, ubichinones and polyphenols may be administered in one pharmaceutical form or in various pharmaceutical forms (such as in two different pharmaceutical forms). If two or more pharmaceutical forms are used, the pharmaceutical forms are preferably consumed by the human(s) at the same time.
At the same time means that the pharmaceutical forms (A) and (B) are orally consumed within a time period of 1 day, preferably within a time period of 1h, more preferably within a time period of 5 minutes, even more preferably within a time period of 1 minute.

The pharmaceutical form(s) comprise the compounds(s) selected from the group consisting of polyunsaturated fatty acids, esters of polyunsaturated fatty acids such as ethyl esters, mono-, di- and triglyceridesters, and fish oil; and/ or the compound(s) selected from the group consisting of carotenoids, water-soluble vitamins, fat-soluble vitamins, ubichinones and polyphenols and optionally a suitable excipient and/or carrier.

The term pharmaceutical form comprises a gelcap, capsule, powder, solid tablet (coated or non-coated), syrup, drink ampoules and sachets/pouches preferably a tablet or capsule such as a hard (shell) gelatin capsule. Suitable excipient and/or carriers include maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and the like (including mixtures thereof). Preferred carriers include calcium carbonate, magnesium stearate, maltodextrin, and mixtures thereof. The various ingredients and the excipient and/or carrier are mixed and formed into the desired form using conventional techniques. The tablet or capsule of the present invention may be coated with an enteric coating that dissolves at a pH of about 6.0 to 7.0. A suitable enteric coating that dissolves in the small intestine but not in the stomach is cellulose acetate phthalate. Further details on techniques for formulation for and administration may be found in the latest edition of Remington's Pharmaceutical Sciences (Maack Publishing Co., Easton, PA).

Thus the invention also relates to a kit for oral intake comprising separate containers packed together in a unitary form comprising
i) a pharmaceutical form (A) comprising at least one compound selected from the group consisting of polyunsaturated fatty acids, esters of polyunsaturated fatty acids such as ethyl esters, mono-, di- and triglyceridesters, and fish oil; and/ or a compound selected from the group consisting of carotenoids, water-soluble vitamins, fat-soluble vitamins, ubichinones and polyphenols with the definitions and preferences as outlined above and optionally a suitable excipient and/or carrier and,
ii) a pharmaceutical form (B) different from i) comprising at least one compound selected from the group consisting of polyunsaturated fatty acids, esters of polyunsaturated fatty acids such as ethyl esters, mono-, di- and triglyceridesters, and fish oil; and/ or a compound selected from the group consisting of carotenoids, water-soluble vitamins, fat-soluble vitamins, ubichinones and polyphenols with the definitions and preferences as outlined above and optionally a suitable excipient and/or carrier and
wherein i) and ii) are individually contained then packaged together in a unitary form and with the proviso that at least one compound selected from the group consisting of polyunsaturated fatty acids, esters of polyunsaturated fatty acids such as ethyl esters, mono-, di- and triglyceridesters, and fish oils and at least one compound selected from the group consisting of carotenoids, water-soluble vitamins, fat-soluble vitamins, ubichinones and polyphenols is present either in the pharmaceutical form (A) or (B) and whereas the total amount of component(s) selected from the group consisting of carotenoids, water-soluble vitamins, fat-soluble vitamins, ubichinones and polyphenols in the pharmaceutical forms (A) and (B) is at least 40 weight-%, preferably at least 50 weight-%, based on the total weight of component(s) selected from the group consisting of polyunsaturated fatty acids, esters of polyunsaturated fatty acids such as ethyl esters, mono-, di- and triglyceridesters, and fish oil in the pharmaceutical forms (A) and (B) based on the weight of the pure compounds.

In another embodiment the invention relates to a personal care kit comprising separate containers packaged together in a unitary form comprising:
i) a composition suitable for oral consumption according to the invention and
ii) a product suitable for topical application to the skin wherein
i) and ii) are individually contained then packaged together in a unitary form.

The kits of the present invention are useful for supporting a healthy skin appearance and beauty, for supporting skin nourishment from inside the body via the blood stream (systemically) an in the case of the personal care kit also from outside the body via topical application of a cosmetic composition, for fostering hydration, for moisturizing the skin, for supporting the skin barrier function, for providing protection against oxidative stress, for providing protection against UV-radiation and/or for providing a general anti-aging effect. The kits of the present invention are in particular-suitable for promoting skin repair and/or regeneration upon healing of injuries and/or for promoting the physiological renewal process and thus for promoting an optimal health, a natural radiance and glow and/or a beautiful look of the skin.

The kits of the present invention are preferably presented to a user or potential user (hereinafter "users") in association with information which informs such users that use of the kit will provide one or more benefits, including, but not limited to, supporting a healthy skin appearance and beauty, supporting skin nourishment from inside the body via the blood stream (systemically), fostering hydration, moisturizing the skin, supporting the skin barrier function providing protection against oxidative stress, providing protection against UV-radiation and/or providing a general anti-aging effect, in particular for promoting skin repair and/or regeneration upon healing of injuries and/or for promoting the physiological renewal process and thus for promoting an optimal health, a natural radiance and glow and/or a beautiful look of the skin. Such information may also include instructions for use to obtain such benefits, e.g., including a detailed description of the mode of application such as described above. By "in association with information" it is meant that the information is either directly printed on the packaging of the kit itself (including direct printing on the container per se or indirectly via a label or the like affixed to the container), or presented in a different manner including, but not limited to, a brochure, print advertisement, electronic advertisement and/or other advertisement, so as to communicate the information to a consumer of the composition. Such information may accordingly comprise words, pictures, and the like.

The term topical skin care product refers to cosmetic compositions that can be topically applied to mammalian keratinous tissue.

The term "cosmetic preparation" or "cosmetic composition" as used in the present application refers to cosmetic compositions as defined under the heading "Kosmetika" in Römpp Lexikon Chemie, 10th edition 1997, Georg Thieme Verlag Stuttgart, New York.

Preferably, the cosmetic or pharmaceutical compositions of the present invention are topical compositions in the form of a suspension or dispersion in solvents or fatty substances, or alternatively in the form of an emulsion or micro emulsion (in particular of O/W or W/O type, O/W/O or W/OlW-type), PET-emulsions, multiple emulsions, bickering emulsions, hydrogels, alcoholic gels, lipogels, one or multiphase solutions or a vesicular dispersion and other usual compositions, which can also be applied by pens, as masks or as sprays. The emulsions can also contain anionic, nonionic, cationic or amphoteric surfactant(s).

Preferred cosmetic compositions according to the invention are skin care preparations, decorative preparations, light protection preparations and functional preparations.

Examples of skin care preparations are, in particular, body oils, body lotions, body gels, treatment creams, skin protection ointments, shaving preparations, such as shaving foams or gels, skin powders such as baby powder, moisturizing gels, moisturizing sprays, revitalizing body sprays, cellulite gels face and/or body moisturizers, facial and/or body cleansers anti acne preparations and peeling preparations.

Examples of decorative preparations are in particular lipstick, eye shadow, mascaras, dry and moist make-up, rouge, powders, and suntan lotions.

Examples of functional preparations are cosmetic or pharmaceutical compositions containing active ingredients such as hormone preparations, vitamin preparations, vegetable extract preparations, anti-ageing preparations, and antimicrobial (antibacterial or antifungal) preparations without being limited thereto.

Cosmetic compositions in accordance with the invention can be in the form of a liquid, lotion, a thickened lotion, a gel, a cream, a milk, an ointment, a paste, a powder, a make-up, or a solid tube stick and can be optionally be packaged as an aerosol and can be provided in the form of a mousse such as a aerosol mousse, a foam or a spray foams, sprays, sticks, a gel, a plaster, a powder, a cleanser, a soap or aerosols or wipes.

In accordance with the present invention, the product suitable for topical application to the skin contains at least one cosmetically active ingredient in particular for skin lightening; tanning prevention; treatment of hyperpigmentation; preventing or reducing acne, wrinkles, lines, atrophy and/ or inflammation; as well as topical anesthetics; antimicrobial and/ or antifungal agents; chelators and/ or sequestrants; anti-cellulites agents and/ or sunscreening additives and carriers and/or excipients or diluents conventionally used in topical compositions. If nothing else is stated, the excipients, additives, diluents, etc. mentioned in the following are suitable for cosmetic compositions. The necessary amounts of the cosmetic and dermatological adjuvants and additives can, based on the desired product, easily be determined by the skilled person.

In any embodiment of the present invention, however, the cosmetically active ingredients useful herein can be categorized by the benefit they provide or by their postulated mode of action on the skin. However, it is to be understood that the actives useful herein can in some instances provide more than one benefit or operate via more than one mode of action.

Examples of cosmetically active ingredients to be used in the cosmetic compositions according to the invention comprise peptides (e.g., Matrixyl^{™} [pentapeptide derivative]), oligopeptides, wax-based synthetic peptides (e.g., octyl palmitate and tribehenin and sorbitan isostearate and palmitoyl-oligopeptide), iodopropyl butylcarbamate, glycerol, urea, guanidine (e.g. amino guanidine); vitamins and derivatives thereof such as vitamin C (ascorbic acid), vitamin A (e.g., retinoid derivatives such as retinyl palmitate or retinyl propionate), vitamin E (e.g., tocopherol acetate), vitamin B₃ (e.g. niacinamide) and vitamin B₅ (e.g. panthenol), vitamin B₆ and vitamin B₁₂, biotin, folic acid; anti-acne actives or medicaments (e.g. resorcinol, salicylic acid, and the like); antioxidants (e.g. phytosterols, lipoic acid); flavonoids (e.g. isoflavones, phytoestrogens); skin soothing and healing agents such as aloe vera extract, allantoin and the like; agents suitable for aesthetic purposes such as essential oils, fragrances, skin sensates, opacifiers, aromatic compounds (e.g., clove oil, menthol, camphor, eucalyptus oil, and eugenol), desquamatory actives, hydroxy acids such as AHA acids, poly unsaturated fatty acids, radical scavengers, farnesol, antifungal actives in particular bisabolol, alkyldiols such as 1,2-pentanediol, hexanediol or 1,2-octanediol, phytol, polyols such as phytanetriol, ceramides and pseudoceramides, amino acids, protein hydrolysates, polyunsaturated fatty acids, plant extracts like kinetin, DNA or RNA and their fragmentation products, carbohydrates, conjugated fatty acids, carnitin, carnosine, biochinonen, phytofluen, phytoen, and their corresponding derivatives, co-enzyme Q10/ubiquinone), anti-oxidants [preferably epigallocatechin galate (EGCG), hydroxytyrolsol, or olive extract] without being limited thereto.

The topical cosmetic compositions of the invention can also contain usual cosmetic adjuvants and additives, such as preservatives/ antioxidants, fatty substances/ oils, water, organic solvents, silicones, thickeners, softeners, emulsifiers, sunscreens, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, e.g. those suited for providing a photo-protective effect by physically blocking out ultraviolet radiation, or any other ingredients usually formulated into cosmetic compositions. Such cosmetic ingredients commonly used in the skin care industry, which are suitable for use in the compositions of the present invention are e.g. described in the CTFA Cosmetic Ingredient Handbook, Second Edition (1992) without being limited thereto. The necessary amounts of the cosmetic and dermatological adjuvants and additives can, based on the desired product, easily be chosen by a skilled artisan in this field and will be illustrated in the examples, without being limited hereto. The usual cosmetic adjuvants and additives such as emulsifiers, thickeners, surface active ingredients and film formers can show synergistic which can be determined by the expert in the field with normal trials, or with the usual considerations regarding the formulation of cosmetic composition.

The amount of the product suitable for topical application to the skin, which is to be applied, depends on the concentration of the active ingredients in the product and the desired cosmetic effect. For example, application can be such that a creme is applied to the skin. A crème is usually applied in an amount of 2 mg creme/cm² skin. The amount of the product suitable for topical application to the skin which is applied to the skin is, however, not critical, and if with a certain amount of applied product suitable for topical application to the skin the desired effect cannot be achieved, a higher concentration of the product suitable for topical application to the skin can be used e.g. by applying more of the product suitable for topical application to the skin or by applying product suitable for topical application to the skin which contain more of active ingredients.

According to the invention, in combination with the oral consumption of a composition according to the invention, a product suitable for topical application is chronically applied to the skin.
By "chronic topical application" is meant continued topical application of the composition at least once a day over an extended period during the subject's lifetime, preferably once a day for a period of at least about a week, more preferably once a day for a period of at least about one month, even more preferably once a day for at least about three months, even more preferably once a day for at least about six months, and still more preferably once a day for at least about one year. While benefits are obtainable after various maximum periods of use (e. g. five, ten or twenty years), it is preferred that chronic application continues throughout the subject's lifetime. Typically applications would be on the order of at least once a day over such extended periods, however application rates can vary from about once per week up to about three times per day or more. A wide range of quantities of the topical product of the present invention can be employed to provide a keratinous tissue appearance and/or feel benefit. Quantities of the topical product that are typically applied per application will vary according to the user's level of regulation desired, e.g., in light of the level of skin damage present or expected to occur. For example, the topical cosmetic composition of the present invention useful for regulating skin condition is preferably practiced by applying a composition in the form of a skin lotion, cream, gel, foam, emulsion, spray, conditioner, tonic, cosmetic, lipstick, foundation, nail polish, after-shave, or the like which is intended to be left on the skin or other keratin structure for some aesthetic, prophylactic, therapeutic or other benefit (i.e., a "leave-on" composition). After applying the composition to the skin, it is preferably left on the skin for a period of at least about 15 minutes, more preferably at least about 30 minutes, even more preferably at least about 1 hour, most preferably for at least several hours, e. g. , up to about 12 hours. Any part of the external portion of the face, can be treated, e.g., face, lips, under-eye area, eyelids, scalp, neck, torso, arms, hands, legs.

The following examples are provided to further illustrate the processes and compositions of the present invention. These examples are illustrative only and are not intended to limit the scope of the invention in any way.

### Example 1 "Ideal skin formula I" for oral consumption

Lecithin is dissolved in the ROPUFA® 75 n-3 '75'EE (ethylester, containing min. 75% n-3 fatty acid ethyl ester, stabilized with mixed tocopherol, ascorbyl palmitate and rosemary extracts; commercially available from DSM Nutritional Products AG, Kaiseraugst, Switzerland) and the beta-Carotene 30% FS, Lutein 20% FS (extracted from Tagetes erecta in Corn Oil and stabilized with DL-alpha-Tocopherol), redivivo^{™} (lycopene) 10% FS (in cornoil, stabilized with DL-alpha-Tocopherol) (commercially available from DSM Nutritional Products AG, Kaiseraugst, Switzerland) are added, vitamin C, Hidrox®, Biotin as (Co-N'Zyme™ R commercially available from DSM Nutritional Products AG, Kaiseraugst, Switzerland) and CoEnzyme Q10 (as ALL-Q 10% CWS/S commercially available from DSM Nutritional Products AG, Kaiseraugst, Switzerland) are mixed in a tumbler mixer for 5 minutes. This dry powder mix is dispersed in the oily mixture of ROPUFA, carotene and lecithin and then encapsulated in capsules according to a commonly applied procedure.

| | |
|---|---|
| Beta-carotene | 3 mg (= 10 mg beta-Carotene 30 % FS) |
| Lycopene (Redivio) | 3 mg (= 30 mg redivivo^{™} (lycopene) 10 % FS) |
| Lutein | 3 mg (= 15 mg Lutein 20 % FS) |
| D/L-alpha-Tocopherolacetate | 75 mg |
| Vitamin C | 75 mg (as fine powder) |
| ROPUFA® 75 n-3 '75'EE | 400 mg |
| Hidrox® | 100 mg (provides 6 mg polyphenols and 2.5 mg hydroxytyrosol) |
| Biotin (**Co-N'Zyme™ R**) | 150 µg |
| CoEnzyme Q10 (All-Q) | 5 mg (=50 mg **ALL-Q** 10 % CWS/S) |
| Lecithin as mixed soybean phosphatides | 25 mg |

One capsule is taken per day together with a meal.

### Example 2 "Ideal skin formula II" for oral consumption

Lecithin is dissolved in the ROPUFA® 75 n-3 '75'EE (ethylester, containing min. 75% n-3 fatty acid ethyl ester, stabilized with mixed tocopherol, ascorbyl palmitate and rosemary extracts; commercially available from DSM Nutritional Products AG, Kaiseraugst, Switzerland) and the beta-Carotene 30% FS, Optisharp^{™} 20% FS (in cornoil stabilized with DL-alpha-Tocopherol) are added. Teavigo, vitamin C, Hidrox®, Biotin as (Co-N'Zyme™ R commercially available from DSM Nutritional Products AG, Kaiseraugst, Switzerland) and CoEnzyme Q10 (as ALL-Q 10% CWS/S commercially available from DSM Nutritional Products AG, Kaiseraugst, Switzerland) are mixed in a tumbler mixer for 5 minutes. This dry powder mix is dispersed in the oily mixture of ROPUFA, carotene and lecithin and then encapsulated in capsules according to a commonly applied procedure.

| | |
|---|---|
| Beta-carotene | 6 mg (= 20 mg beta-Carotene 30 % FS) |
| Zeaxanthin | 2.0 mg (= 10 mg Optisharp^{™} 20 % FS) |
| D/L-alpha-tocopherolacetate | 300 mg |
| Vitamin C | 100 mg |
| TEAVIGO ™ | 150 mg |
| ROPUFA® 75 n-3 '75'EE | 400 mg |
| Hidrox® | 100 mg (provides 6 mg polyphenols and 2.5 mg hydroxytyrosol) |
| Biotin (Co-N'Zyme™ R) | 300 µg |
| CoEnzyme Q10 (All-Q) | 20 mg (=200 mg ALL-Q 10 % CWS/S) |
| Lecithin as mixed soybean phosphatides | 40 mg |

One capsule is taken per day together with a meal.

### Example 3 "A kit for oral consumption"

The liquid active ingredients beta-carotene, zeaxanthin, DL-alpha-tocoherol-acetate, and ROPUFA 75 n-3 '75'EE can be provided within a capsule whereas the solid ingredients vitamin C, Hidrox, Biotin and CoQ10 are provided in the form of a tablet. The tablet may be prepared with commonly known tabletting excipients such as dry binders e.g. microcrystalline cellulose, lactose, other carbohydrates or carbohydrate derivatives like starch, sorbitol, mannitol dextrins etc. A disintegrant like croscarmelllose or crospovidone may be added in an appropriate amount, as well as a lubricant like Mg-stearate or a similar compound, a behenate polyethyleneglycol, or any other lubricant.

### Capsule

| | |
|---|---|
| Beta-carotene | 6 mg (= 20 mg beta-Carotene 30 % FS) |
| Zeaxanthin | 2.0 mg (= 10 mg Optisharp^{™} 20 % FS) |
| D/L-alpha-tocopherol-acetate | 300 mg |
| ROPUFA® 75 n-3 '75'EE | 400 mg |
| Biotin (**Co-N'Zyme™ R**) | 300 µg |
| Lecithin as mixed soybean phosphatides | 40 mg |

This oily mix is encapsulated in capsules according to a commonly applied procedure.

### Tablet

| | |
|---|---|
| Vitamin C | 110 mg (as Ascorbic Acid 90% Granulation) |
| TEAVIGO TG™ | 150 mg |
| Hidrox® | 100 mg (provides 6 mg polyphenols and 2.5 mg hydroxytyrosol) |
| Biotin (Co-N'Zyme™ R) | 300 µg |
| CoEnzyme Q10 (All-Q) | 20 mg (=200 mg ALL-Q 10 % CWS/S) |
| Microcrystalline cellulose | 300 mg |
| Lactose | 300 mg |
| Crospovidone | 35 mg |
| Mg-Stearate | 24.7 mg |

Biotin and microcrystalline cellulose is mixed in a tumbler mixer for 10 min. Then, lactose is added and the composition mixed for 10 min again. Vitamin C, Teavigo TG, Hidrox, CoQ10 and Crospovidone are combined with the other ingredients and mixed for 10 min. Finally, Mg-stearate is added to the other components and mixed for another 2 min. The mixture is compressed to tablets.

In order to provide the kit for oral intake according to the invention, the tablet and the capsule are individually packed in separate containers and then packed together in a unitary form.

Per day one capsule and one tablet are taken at the same time together with a meal.

### Example 3 "A personal care kit"

A capsule as described in example 1 or 2 is used together with an anti-ageing cream.
The anti-aging cream containing the ingredients indicated below can be prepared in a manner known per se.

### Anti-aging cream

| O/W emulsion with 4-Phenyl-butyric acid 1,5-dimethyl- | |
|---|---|
| **Ingredients** | **% (w/w)** |
| Glyceryl Myristate | 4.00 |
| Cetyl Alcohol | 2.00 |
| Steareth-2 | 2.00 |
| Steareth-21 | 2.00 |
| Isopropyl Myristate | 5.00 |
| Caprylic/Capric Triglyceride | 8.00 |
| BHT | 0.05 |
| Dimethicone | 2.00 |
| Phenoxyethanol & Methylparaben & Ethylparaben & Butylparaben & Propylparaben & Isobutylparaben | 0.80 |
| CoEnzyme Q10 (All-Q) | 0.50 |
| Water | Ad 100 |
| Xanthan Gum | 0.50 |
| Disodium EDETA | 0.10 |
| Propylene Glycol | 4.00 |

In order to provide the personal care kit according to the invention, the capsule and the anti-ageing cream are individually packed in separate containers and then packed together in a unitary form.
The anti-ageing cream is applied once a day. One capsule is taken per day together with a meal.

## Claims

1. A composition for oral intake comprising
i) at least a compound selected from the group consisting of polyunsaturated fatty acids, esters of polyunsaturated fatty acids such as ethyl esters, mono-, di- and triglyceridesters, and fish oil; and
ii) at least a compound selected from the group consisting of carotenoids, water-soluble vitamins, fat-soluble vitamins, ubichinones and polyphenols,
whereas the total amount of component(s) ii) in the composition is at least 40 weight-%, preferably at least 50 weight-%, based on the total weight of component(s) i) and based on the weight of the pure compounds.

2. The composition according to claim 1, wherein the component i) is essentially a mixture of eicosapentaenoic acid, docosahexaenoic acid and their esters.

3. The composition according to claim 2, wherein the esters are the ethyl esters.

4. The composition according to claim 1, wherein the carotenoids are selected from the group consisting of β-carotene, lutein, zeaxanthin, and lycopene and pharmaceutically acceptable derivatives thereof.

5. The composition according to claim 1, wherein the water-soluble vitamins are selected from the group consisting of the B vitamins, vitamin C, biotin and pharmaceutically acceptable salts and derivatives thereof.

6. The composition according to claim 1, wherein the fat-soluble vitamins are selected from the group consisting of vitamin A, vitamin E and pharmaceutically acceptable derivatives thereof.

7. The composition according to claim 1, wherein the ubichinones are selected from the group consisting of coenzyme Q 10, vitamin K and pharmaceutically acceptable derivatives thereof.

8. The composition according to claim 1, wherein the polyphenols are selected from the group consisting of epigallacatechin 3-gallate, hydroxytyrosol, resveratrol, genistein and pharmaceutically acceptable derivatives thereof.

9. A composition for consumption by humans comprising
a) at least a compound selected from the group consisting of polyunsaturated fatty acids, esters of polyunsaturated fatty acids such as ethyl esters, mono-, di- and triglyceridesters, and fish oil, in amount in the range of from 0.50 mg to 120 mg per kg bodyweight of said humans, preferably from 5 mg to 30 mg per kg bodyweight of said humans; and at least one of the components b) to m) selected from
b) β-carotene, in an amount in the range of from 0.0014 to 0.7 mg per kg bodyweight, preferably from 0.01 and 0.5 mg per kg bodyweight of said humans;
c) lycopene, preferably in an amount in the range of from 0.0014 to 0.6 mg, more preferably between 0.007 to 0.35 mg per kg bodyweight of said humans;
d) lutein, in an amount in the range of from 0.0014 to 0.86 mg, preferably between 0.014 to 0.5 mg per kg bodyweight of said humans;
e) Zeaxanthin, in an amount in the range of from 0.0014 to 0.28 mg, preferably between 0.028 to 0.1 mg per kg bodyweight of said humans;
f) vitamin E, in an amount in the range of from 0.14 mg and 30 g, preferably between 0.2 and 7 mg per kg bodyweight of said humans;
g) vitamin C, in an amount in the range of from 0.71 mg and 70 g, preferably between 2.5 mg and 20 mg per kg bodyweight of said humans;
h) epigallocatechin gallate, in an amount in the range of from 0.5 to 8.5 mg, preferably 2.0 to 4.3 mg per kg bodyweight of said humans; and/or
i) hydroxytyrosol, in an amount in the range of from 0.071 to 7.1 mg, preferably 0.71 to 1.42 mg per kg bodyweight of said humans;
j) genistein: in an amount in the range of from 0.014 to 2.14 mg, preferably 0.28 to 0.85 mg per kg bodyweight of said humans;
k) resveratrol: in an amount in the range of from 0.014 to 1.4 mg, preferred preferably 0.071 to 0.71 mg, more preferred from 0.28 to 0.42 mg per kg bodyweight of said humans;
l) biotin, in an amount in the range of from 0.14 µg to 71 µg, preferably 0.28 µg to 28 µg per kg bodyweight of said humans;
m) coenzyme Q 10, in an amount in the range of from 0.014 to 1.4 mg, preferably 0.071 to 0.85 mg per kg bodyweight of said humans;
wherein the total amount of components b) to m) is at least 40 weight-%, preferably at least 50 weight-%, based on the total weight of component(s) a) and based on the weight of the pure compounds.

10. A composition for consumption by humans comprising
i) at least a compound selected from the group consisting of polyunsaturated fatty acids, esters of polyunsaturated fatty acids such as ethyl esters, mono-, di- and triglyceridesters, and fish oil, in amount in the range of from 0.50 mg to 120 mg per kg bodyweight of said humans, preferably from 5 mg to 30 mg per kg bodyweight of said humans; and
ii) at least a polyphenol selected from the group consisting of epigallocatechin gallate, genistein, resveratrol and pharmaceutically acceptable derivatives thereof, in an amount in the range of from 0.014 to 8.5 mg, preferably 0.14 to 4.3 mg per kg bodyweight of said humans; and/or hydroxytyrosol and pharmaceutically acceptable derivatives thereof, in an amount in the range of from 0.014 to 7.1 mg, preferably 0.71 to 1.42 mg per kg bodyweight of said humans,
and/or olive extract in an amount in the range of from 0.014 to 7.1 mg, preferably 0.71 to 1.42 mg per kg bodyweight of said humans;

11. The composition according to claim 10 further comprising lipid soluble and/or water soluble vitamins, co-enzymes, anti-oxidants, carotenoids and/or their esters.

12. Use of the compositions according to any of claims 1 to 12 for supporting a healthy skin appearance and beauty, for supporting skin nourishment from inside the body via the blood stream (systemically), for fostering hydration, for moisturizing the skin, for supporting the skin barrier function, for providing protection against oxidative stress, for providing protection against UV-radiation and/or for providing a general anti-aging effect.

13. Use of the composition according to any of claims 1 to 12 for promoting skin repair and/or regeneration upon healing of injuries and/or for promoting the physiological renewal process.

14. Use of the composition according to any of claims 1 to 12 for promoting an optimal health, natural radiance and glow and/or a beautiful look of the skin.

15. A method of administering an effective amount of
i) at least a compound selected from the group consisting of polyunsaturated fatty acids, esters of polyunsaturated fatty acids such as ethyl esters, mono-, di- and triglyceridesters, and fish oil; and
ii) at least a compound selected from the group consisting of carotenoids, water-soluble vitamins, fat-soluble vitamins, ubichinones and polyphenols,
whereas the total amount of component(s) ii) in the composition is at least 40 weight-%, preferably at least 50 weight-%, based on the total weight of component(s) i) and based on the weight of the pure compounds to humans in order to support a healthy skin appearance and beauty, to support skin nourishment from inside the body via the blood stream (systemically), to foster hydration, to support moisturizing the skin, to support the skin barrier function, to provide protection against oxidative stress, to provide protection against UV-radiation and/or to provide a general anti-aging effect, in particular to promote skin repair and/or regeneration upon healing of injuries and/or to promote the physiological renewal process and thus to promote an optimal health, a natural radiance and glow and/or a beautiful look of the skin.

16. A kit for oral intake comprising separate containers packed together in a unitary form comprising
(i) a pharmaceutical form (A) comprising at least one compound selected from the group consisting of polyunsaturated fatty acids, esters of polyunsaturated fatty acids such as ethyl esters, mono-, di- and triglyceridesters, and fish oil; and/ or a compound selected from the group consisting of carotenoids, water-soluble vitamins, fat-soluble vitamins, ubichinones and polyphenols with the definitions and preferences as outlined above and optionally a suitable excipient and/or carrier and
(ii) a pharmaceutical form (B) different from i) comprising at least one compound selected from the group consisting of polyunsaturated fatty acids, esters of polyunsaturated fatty acids such as ethyl esters, mono-, di- and triglyceridesters, and fish oil; and/ or a compound selected from the group consisting of carotenoids, water-soluble vitamins, fat-soluble vitamins, ubichinones and polyphenols and optionally a suitable excipient and/or carrier and
wherein i) and ii) are individually contained then packaged together in a unitary form and
with the proviso that at least one compound selected from the group consisting of polyunsaturated fatty acids, esters of polyunsaturated fatty acids such as ethyl esters, mono-, di- and triglyceridesters, and fish oils and at least one compound selected from the group consisting of carotenoids, water-soluble vitamins, fat-soluble vitamins, ubichinones and polyphenols is present either in the pharmaceutical form (A) or (B) and
whereas the total amount of component(s) selected from the group consisting of carotenoids, water-soluble vitamins, fat-soluble vitamins, ubichinones and polyphenols in the pharmaceutical forms (A) and (B) is at least 40 weight-%, preferably at least 50 weight-%, based on the total weight of component(s) selected from the group consisting of polyunsaturated fatty acids, esters of polyunsaturated fatty acids such as ethyl esters, mono-, di- and triglyceridesters, and fish oil in the pharmaceutical forms (A) and (B) based on the weight of the pure compounds

17. A personal care kit comprising separate containers packaged together in a unitary form comprising
i) a composition suitable for oral consumption according to any one of claims 1 to 11,
ii) a product suitable for topical application to the skin
wherein i) and ii) are individually contained then packaged together in a unitary form.

18. A personal care kit according to claim 17, wherein the product suitable for topical application to the skin is a skin care preparation, a decorative preparation, a light protection preparation or a functional preparation.

19. A personal care kit according to claim 17 and 18, wherein the product suitable for topical application to the skin contains at least one cosmetically active ingredient.

20. The invention as particularly described hereinbefore, especially with reference to the Examples.
